# EUROPEAN PATENT SPECIFICATION

(11) **EP 3 300 502 B1**
(45) Date of publication and mention of the grant of the patent: **22.07.2020**
(21) Application number: 16786855.3
(22) Date of filing: 29.04.2016
(51) Int. Cl.: C07H 1/08, C07H 3/06, A61K 31/702, A61P 1/14, C12P 19/14, C08B 37/00

(54) **PREPARATION COMPRISING ARABINOXYLO-OLIGOSACCHARIDES**
ZUBEREITUNG ENTHALTEND ARABINOXYLO-OLIGOSACCHARIDE
PRÉPARATION COMPRENANT DES ARABINOXYLO-OLIGOSACCHARIDES

(30) Priority: 30.04.2015 SE 1550542
(43) Date of publication of application: 04.04.2018
(73) Proprietor: Carbiotix AB, 223 63 Lund (SE)
(72) Inventor: FALCK, Peter, 224 79 Lund (SE)
(74) Representative: AWA Sweden AB
(86) International application number: PCT/SE2016/050377
(87) International publication number: WO 2016/175702

(56) References cited:
- WO-A1-2006/002495
- WO-A1-2014/083166
- WO-A2-2008/087167
- WO-A2-2009/040445
- WO-A2-2009/117790
- WO-A2-2010/088744
- GB-A- 2 464 769
- RANTANEN ET AL: "Preparation of arabinoxylobiose from rye xylan using family 10 Aspergillus aculeatus endo-1,4-@b-d-xylanase", CARBOHYDRATE POLYM, APPLIED SCIENCE PUBLISHERS, LTD. BARKING, GB, vol. 68, no. 2, 16 February 2007 (2007-02-16), pages 350-359, XP005892451, ISSN: 0144-8617, DOI: 10.1016/J.CARBPOL.2006.11.022
- PASTELL, E. ET AL.: 'Step-wise enzymatic preparation and structural characterization of singly and doubly substituted arabinoxylo-oligosaccharides with non-reducing end terminal branches' CARBOHYDR. RES. vol. 343, no. 18, 24 September 2008, pages 3049 - 3057, XP025625793
- IZYDORCZYK, M. S. ET AL.: 'Cereal arabinoxylans: advances in structure and physicochemical properties' CARBOHYDR. POLYM. vol. 28, no. 1, 1995, pages 33 - 48, XP004034428

## Description

### Field of the invention

The present invention relates to methods for producing preparations comprising arabinoxylo-oligosaccharides, which are particularly useful as food or beverage ingredients or as nutritional supplements. Further the invention also relates to the improved generation of arabinoxylo-oligosaccharides in established prebiotic formulations comprising mixtures between xylo-oligosaccharides and arabinoxylo-oligosaccharides.

### Technical Background

Xylan hemicelluloses are the second most abundant biopolymers in the plant kingdom after cellulose. A common feature for all xylans in higher plants are their backbone of β-(1→4)-linked D-xylopyranosyl (Xyl*p*) residues. Xylans containing other sugars than xylose are called heteroxylans and can be divided into glucuronoxylans (GX), found in secondary cell walls of dicot plants, or arabinoxylan (AX), found in the primary cell walls of cereals. AX content and composition in cereals varies with botanical source, cultivar and tissue. Most AX is found in the outer bran tissues (outer- and inner pericarp, testa, nucellar epidermis and associated aleurone layer), although the starchy endosperm also contains a considerable amount. AX can be classified as water extractable (WE-AX) or water-unextractable (WU-AX). Solubility in water is limited by covalent and/or non-covalent linkages to other cell wall components. While easily soluble, AX is weakly bound at the surface.

Generally four main structural elements are present in cereal AX, I) unsubstituted Xyl*p* units (uXyl), II) α-(1→2)-linked L-arabinofuranosyl (Ara*f*) linked to a Xyl*p* unit (mXyl2), III) α-(1→3)-linked Ara*f* linked to a Xyl*p* unit (mXyl3) and IV) double α-(1→2) and α-(1→3)-linked Ara*f* linked to a Xyl*p* unit (dXyl). In addition, galactose and glucuronic acids can be present in AX from the outer grain tissues. Hydroxycinnamic acid derivatives, mainly ferulic acid (FA), can be ester-linked to position O-5 on a few Ara*f* substituents causing oxidative gelation in water (crosslinking of dihydrodiferulic acids) and antioxidant properties. The molar ratio of arabinose and xylose (A/X) in AX is an important characteristic when it comes to enzymatic hydrolysis, solubility and fermentative properties in the gastrointestinal tract (GIT).

Oligosaccharides, which are short saccharide polymers, can be derived by partial hydrolysis of the AX backbone using either thermo/chemical or enzymatic methods. Considering only AX from cereals, two main groups of oligosaccharides can be obtained from enzymatic hydrolysis: xylo-oligosaccharides (XOS) and arabinoxylo-oligosaccharides (AXOS). XOS are xylose oligomers, which are linked by β-(1→4) linkages with the general molecular formula C₅ₙH₈ₙ+2 O₄ₙ+1, where n is the number of xylose units 2-10. The XOS are X₂: xylobiose, X₃: xylotriose, X₄: xylotetraose, X₅: xylopentaose, X₆: xylohexaose, X₇: xyloheptaose, X₈: xylooctaose, X₉: xyloenneaose and X₁₀: xylodecaose. AXOS on the other hand have a XOS as a backbone with at least one Ara*f* group attached as a side chain to one of the xylose units. Depending on how many Ara*f* groups are attached, to which residue, and on the chemical linkage type (1→2) and/or (1→3), many different combinations of AXOS are possible. Arabinoxylan-oligosaccharides (A)XOS comprises a mixture of both xylo-oligosaccharides (XOS) and arabinoxylo-oligosaccharides (AXOS) and is obtained after enzymatic hydrolysis with commercial xylanases.

Xylanases are used for example, in pulp and paper processing, biofuels production, the baking, and the brewing industries and in processing of animal feed. These enzymes are able to hydrolyze the β-(1→4)-xylosidic linkages found in xylan and xylan derived oligosaccharides. Depending on the xylanase used different size of XOS and structures of AXOS can be generated. Family 10 xylanases are known to produce small end products. This is consistent with the production of xylose and X₂ as the main hydrolysis products from AX. The smallest AXOS produced by family 10 xylanases is a tri-saccharide (A³X). Family 11 xylanases have the same catalytic mechanism as does family 10, but activity is generally higher on polymeric substrates than oligomeric and they have higher activity against insoluble substrates compared with family 10. The main hydrolysis products by family 11 xylanases are xylose, X₂ and X₃ while the smallest AXOS is a tetrasaccharide (A³XX).

There is a commercial interest in XOS and AXOS as emerging prebiotics, defined as "a selectively fermented ingredient that results in specific changes in the composition and/or activity of the GI microbiota, thus conferring benefit(s) upon host health". Considerable proofs of these compounds' prebiotic properties are now available based on in vitro and in vivo trials which demonstrated that they fulfil all the criteria for a prebiotic. XOS and AXOS are fermented by the faecal microbiota producing health promoting short chain fatty acids acetate, lactate, propionate and butyrate. Depending on the size of the oligosaccharides, different metabolic acids are produced. The prebiotic effect of XOS and AXOS is tightly linked to the size of the oligosaccharides and the arabinose substitution; a small size is required for most bifidobacteria in order for them to be utilized as a carbon source. The Ara*f* group(s) attached to the AXOS are therefore an important characteristic when it comes to the fermentative properties in the gastrointestinal tract (GIT) since not all XOS utilizing bacteria can utilize AXOS.

Bifidobacteria is considered one of the most important groups of beneficial bacteria due their ability to stimulate immune system development, produce vitamins, inhibit pathogens, reduce ammonia and cholesterol in the blood and help to restore a healthy gut after antibiotic treatment. The ability among bifidobacteria to use XOS and/or AXOS is strain-dependent, meaning that strains can be grouped based on their carbohydrate preference. Bifidobacteria can be clustered into five different groups (I-V) based on their ability to ferment arabinose, xylose, XOS or AXOS. In cluster I, the strains can not use XOS or AXOS. In cluster II strains are able to ferment the arabinose substituents present on AXOS. Cluster III contains strains that are able to ferment the XOS backbone up to xylotetraose, but have a more limited consumption of AXOS. In cluster IV and V the strains have a broad degradation of both XOS and AXOS.

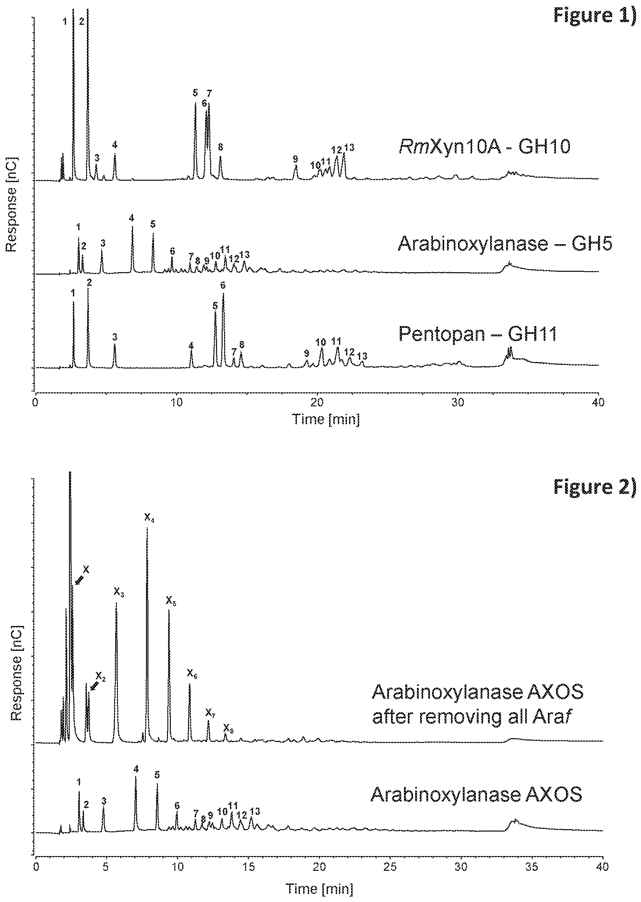

State of the art preparation of prebiotics from AX include (A)XOS products comprising a mixture of both XOS and AXOS obtained by xylanase hydrolysis of AX or AX containing material. In EP 2 265 127, prebiotic (A)XOS preparations are prepared from wheat bran using a family 10 and/or family 11 xylanase. This application is based on a method by Swennen et al. (2006) where the final preparation is a mixture between XOS and AXOS (Swennen et al., 2006, Figure 2a) with relatively little arabinose content indicated by a low A/X ratio 0.25-0.26. These preparation also contains xylose, which is not considered as a prebiotic and would preferably be avoided in the final product. It is therefore clear that all prebiotic preparations from AX described in EP 2 265 127 and scientific literature to date are mixtures of xylose, XOS and AXOS due to the fact that the enzymatic hydrolysis by well-established family 10 and/or 11 xylanases always produces xylose, XOS and AXOS. A limitation with the current technology is therefore to produce pure prebiotic AXOS compositions with no or very little co-formation of xylose and XOS.

The publication "Preparation of arabinoxylobiose from rye xylan using family 10 Aspergillus aculeatus endo-1,4-β-D-xylanase" by Rantanen et al., Carbohydrate Polymers, vol. 68, no 2, (2007), pages 350-359 discloses preparation of the most abundant short AXOS from cereal AX using commercial enzyme Shearzyme® containing *Aspergillus aculeatus GH10* xylanase.

In prior art, arabino-xylooligosaccharide preparations may be found, however there is a need for a more pure, specific preparation of prebiotics from AXOS.

### Summary of the invention

The current invention describes how prebiotic AXOS can be generated from AX using an arabinoxylanase without creating xylose and XOS. The preparations are special in their composition of arabinose containing oligosaccharides without xylose and XOS. Their ability to specifically stimulate certain strains of bifidobacteria make them useful as a more selective prebiotic. Disclosed by way of example only is an AXOS composition comprising at least one arabinose unit linked to one of the xylose units of the backbone, per molecule, wherein the at least one arabinose unit is an α-L-arabinofuranosyl, wherein said composition has an XOS backbone with a degree of polymerization of 1-10. Disclosed by way of example only, the AXOS composition has an average degree of arabinose substitution of 0.3-0.6. Disclosed by way of example only, AXOS composition has an average degree of arabinose substitution of 0.2-0.7. The application of the pure AXOS is to selectively stimulate certain groups of bifidobacteria. Such preparations can be used in food or beverage ingredients or as nutritional supplements with or without added bifidobacteria. Disclosed by way of example only, the AXOS composition selectively is adapted to stimulate the growth of *Bifidobacterium spp.* In another embodiment the *Bifidobacterium spp* belong to strains adapted to ferment AXOS or the arabinose substituents on the oligosaccharides. Disclosed by way of example only, the *Bifidobacterium spp* is selected from the group consisting of *Bifidobacterium adolescentis, Bifidobacterium longum, Bifidobacterium catenulatum, Bifidobacterium animalis, Bifidobacterium pseudolongum, Bifidobacterium gallicum Bifidobacterium lactis, Bifidobacterium infantis, Bifidobacterium bifidum, Bifidobacterium angulatum or Bifidobacterium breve.* Disclosed by way of example only is improved generation of AXOS in established prebiotic (A)XOS formulations comprising mixtures between XOS and AXOS. Disclosed by way of example only is a synbiotic preparation comprising an AXOS composition, further comprising a *Bifidobacterium spp.* In another embodiment the synbiotic preparation is for the treatment of improving gastrointestinal problems. Disclosed by way of example only, the synbiotic preparation is for use as an ingredient in a product selected from the group consisting of food, feed, beverages or nutritional supplements. Disclosed by way of example only is an AXOS composition or a synbiotic preparation comprising AXOS composition, for use in the treatment of improving gastrointestinal problems.

Disclosed by way of example only is selective prebiotics for certain groups of intestinal bacteria belonging to the group of bifidobacteria adapted to ferment AXOS or the arabinose substituents attached to the AXOS molecule(s). Disclosed by way of example only, obtained AXOS, can be used to selectively stimulate the growth of bifidobacteria over other groups of intestinal bacteria that normally can use XOS. Disclosed by way of example only, strains from a cluster selected from the group consisting of II, III, IV and V are selectively stimulated with preparations containing only AXOS. Disclosed by way of example only, the strains of bifidobacteria are selected from the group consisting of *Bifidobacterium longum* subsp. *longum* DSMZ 20219 (Cluster 2), *Bifidobacterium adolescentis* DSMZ 20083 (Cluster 3), *Bifidobacterium longum* subsp. *longum* CCUG 15137 (Cluster 4) or *Bifidobacterium catenulatum* DSMZ 16992 (Cluster 5).

The present invention implies that various starting points and hence various starting material may be used in a process for producing an AXOS composition according to the present invention. In one embodiment endosperm AX is used as starting material. In another embodiment bran is used as a starting material. Various kinds of flour fractions or bran are thinkableas starting material. In another more specific embodiment the bran starting material is selected from the group of cereals such as rye, maize, millets, rice, barley, oat or wheat but not limited to these. Other possible starting materials are pseudocereals such as, but not limited to, quinoa, amaranth or buckwheat. The starting material is flour or bran from any of the above plants. In one embodiment the starting material is flour comprising endosperm AX.

Therefore, an aspect of the present invention relates to a process for producing an AXOS composition from flour comprising the steps of:
A. extracting and isolating an endosperm arabinoxylan fraction from flour;
B. optionally removing starch and proteins from the obtained product of step A;
C. optionally treatment of the endosperm arabinoxylan of step A or product of step B with arabinofuranosidases, preferably one able to remove α-(1→3)-linked L-arabinofuranosyl at double substituted β-(1→4)-linked D-xylopyranosyl units (dXyl);
D. adding an arabinoxylanase to the obtained product of step A, step B or step C; and
E. drying the obtained material of step D.
In one embodiment step C comprise an addition of weak acid. In one embodiment, when the starting material is flour, the A/X ratio is in the interval of 0.2-0.7 preferably 0.28-0.65, preferably 0.35-0.50, preferably 0.38-0.45 preferably 0.4. In one embodiment the invention comprise a step where endosperm AX is enzymatically treated with an arabinofuranosidase. In one embodiment the arabinofuranosidase is selected from the group Arabinoxylan arabinofuranohydrolases, preferably selected from the group consistng of arabinoxylan arabinofuranohydrolase-D3 or arabinoxylan arabinofuranohydrolase-m2,3. The purpose of enzymatical treatment being to remove a fraction of the Ara*f* groups, in order to improve the yield of AXOS. In another embodiment, the present invention is a process for producing an AXOS composition from bran comprising the steps of:
A'. removal of starch and proteins from the bran;
B'. recovery of a solid phase from A';
C'. treating the solid phase with alkaline solution, alkaline and peroxide solution or treating the solid phase with heat to provide a soluble phase;
D'. neutralizing the soluble phase comprising arabinoxylan of C' and recovery of said soluble phase comprising arabinoxylan;
E'. removing arabinose from the arabinoxylan containing soluble phase in step D' using arabinofuranosidases or a weak acid solution to obtain a molar ratio of arabinose to xylose of 0.2-0.7, preferably 0.35-0.5, preferably 0.38-0.45, preferably 0.4;
F'. separation to recover the arabinoxylan obtained from step E', preferably by precipitation or membrane separation;
G'. adding an arabinoxylanase to the arabinoxylan from step F'; and
H'. drying the obtained material of step G'.

In one embodiment the A/X ratio is in the interval of 0.2-0.7 preferably 0.28-0.65, preferably 0.35-0.5, preferably 0.38-0.45 preferably 0.4. In another embodiment the A/X ratio is 0.4. In yet another embodiment the invention is a process for producing an AXOS composition, wherein the AXOS composition is produced using an arabinoxylan specific endoxylanase. In another, more specific embodiment the invention is a process for producing an AXOS composition, wherein the arabinoxylan specific endoxylanase is arabinoxylanase.

Further, in another embodiment, the invention is a process for producing an AXOS composition wherein the step C' includes an optional treatment with arabinofuranosidases to increase the yield of AXOS of 10-100%, more preferred of 50-100%, even more preferred of 70-100%, yet even more preferred of 85-100% and most preferred 100%. In one embodiment the increased yield is of 95-99%.

When preparing an AXOS composition from bran AX additional steps are possible. In one embodiment, the invention relates to a process for producing an AXOS composition, wherein the step A' includes removal of starch and proteins with amylases and proteases respectively. In another embodiment the invention relates to a process for producing an AXOS composition, wherein the step C' includes extraction with alkali and peroxide, with optionally using other means of extraction. Various means of heat treatment are possible in step C'. In one embodiment the step C' includes steam treatment to increase the water soluble AX content. In another embodiment the step C' includes pressurised water treatment. In yet another embodiment the invention relates to a process for producing an AXOS composition, wherein the step E' includes an optional treatment to increase the yield of AXOS. In one embodiment the step E' includes an optional treatment with arabinofuranosidases. In another embodiment step E' includes an optional treatment with a weak acid solution, such as, but not limited to, inorganic acids, preferably hydrochloric acid, preferably sulfuric acid, preferably phosphoric acid or preferably nitric acid.

Another aspect of the present invention relates to use of an arabinoxylanase to improve the generation of AXOS in XOS and AXOS containing preparations. In one embodiment the invention relates to use of an arabinoxylanase to improve the generation of AXOS in (A)XOS, wherein the preparation of XOS and AXOS is prepared using a family 10 or 11 xylanase.

One aspect of the present disclosure relates to an arabinoxylo-oligosaccharide composition comprising at least one arabinose unit linked to one of the xylose units of the backbone, per molecule, wherein the at least one arabinose unit is an α-L-arabinofuranosyl, wherein said composition has a xylo-oligosaccharide backbone with a degree of polymerization of 1-10, wherein the composition comprise at most 10 % monosaccharides and/or at most 10 % xylooligosaccharides.

In one embodiment, the composition according to the present disclosure may comprise monosaccharides present in an amount of at most 20%, at most 15%, at most 10%, at most 8%, at most 5%. at most 4%, at most 3 %, at most 2%, at most 1.6 %, at most 1%, at most 0.1%, or at most 0.01%. In one embodiment the composition according to the present invention may comprise monosaccharides present in an amount of 0.01-20%, 0.05-10%, 0.01-5%, 0.05-5%, 0.05-2%, 0.01-0.1%, 0.01-1%, 0.05-1.8% or 0.05-1.5%.

In one embodiment, the composition according to the present disclosure may comprise xylooligosaccharides present in an amount of at most 20%, at most 15%, at most 10%, at most 8%, at most 5%. at most 4%, at most 3%, at most 2%, at most 1.6 %, at most 1% at most 0.1%, or at most 0.01%. In one embodiment the composition according to the present invention may comprise xylooligosaccharides present in an amount of 0.01-20%, 0.05-10%, 0.01-5%, 0.05-5%, 0.05-2%, 0.01-0.1%, 0.01-1%, 0.05-1.8%, or 0.05-1.5%. In one embodiment the monosaccharides may comprise arabinose. In one embodiment the monosaccharides may comprise xylose. In one embodiment the amount of monosacharides and/or xylooligosaccharides herein is based on the dry weight% of the preparation.

Yet another aspect relates to use of an arabinoxylanase to improve the generation of arabinoxylo-oligosaccharides in xylo-oligosaccharides and arabinoxylo-oligosaccharides comprising preparations. In one embodiment the preparation of xylo-oligosaccharides and arabinoxylo-oligosaccharides is prepared using a family 11 xylanase. In another the arabinoxylanase is a xylanase beloning to glycoside hydrolase family 5. In another embodiment the arabinoxylo-oligosaccharides are generated from a cereal fiber. In another embodiment, use of an arabinoxylanase according to the present invention comprises the steps of:
A'. removing starch and optionally proteins from a cereal fiber;
B'. recovering a solid phase from A';
C'. treating the solid phase from step B' with a xylanase able to hydrolyze water-insoluble arabinoxylan;
D'. adding an arabinoxylanase able to hydrolyze highly substituted arabinoxylan to step C' in order to improve the generation of arabinoxylo-oligosaccharides;
E'. recovering of said soluble phase from step D' comprising the oligosaccharides;
F'. optionally purifying said soluble phase from step E';
G'. concentrating or drying of soluble phase from step F';
In another embodiment the cereal fiber is derived from rye, maize, millets, rice, barley, oat or wheat.

### Short description of the drawings

Figure 1. AXOS generated by an arabinoxylanase are different from the XOS and AXOS mixtures obtained by family 10 and 11 xylanases indicated by the different products obtained as seen by the different retention times (Table 1).
Figure 2. Lower chromatogram: Major AXOS generated from wheat endosperm AX (peaks 1-13) and upper chromatogram: arabinofuranosidase treated sample to expose the xylose and XOS backbone. X: xylose, X₂: xylobiose, X₃: xylotriose, X₄: xylotetraose, X₅: xylopentaose, X₆: xylohexaose, X₇: xyloheptaose and X₈: xylooctaose.
Figure 3. Lower chromatogram: Major AXOS generated from rye endosperm AX (peaks 1-13) and upper chromatogram: HCI treated sample to expose the xylose and XOS backbone. X: xylose, X₂: xylobiose, X₃: xylotriose, X₄: xylotetraose, X₅: xylopentaose, X₆: xylohexaose, X₇: xyloheptaose, X₈: xylooctaose, X₉: xyloenneaose and X₁₀: xylodecaose.
Figure 4. Optimal yield of arabinoxylanase AXOS are in the range of 0.35-0.61 as indicated by the highest yield at 0.43.
Figure 5. Upper chromatogram: Improved generation of AXOS (white arrows) in XOS and AXOS containing mixtures with addition of an arabinoxylanase as indicated as a shift in retention times towards shorter oligosaccharides and disappearance of AXOS peaks (black arrows) in original composition (lower chromatogram).
Figure 6. *Bifidobacterium adolescentis* utilization of arabinoxylanase derived AXOS from rye endosperm AX as indicated by the disappearing peaks 1- 6.
Figure 7. *Lactobacillus brevis* does not utilize arabinoxylanase derived AXOS from rye endosperm AX as indicated by the remaining peaks 1- 6.

### Detailed description of the invention

The present invention is in accordance with the appended claims.

Arabinoxylanases are unique in their specificity for AX since they do not attack unsubstituted xylans. The oligosaccharides generated by these enzymes contain at least one (1→3) Ara*f* group linked to a reducing end Xyl*p* unit. This group of enzymes have not previously been used or considered in the production of prebiotic AXOS from AX or AX containing materials. In the present invention an arabinoxylanase is used to produce AXOS from AX containing materials. These AXOS preparations obtained by the arabinoxylanase are unique prebiotics in their AXOS composition and lack of xylose and XOS. Comparison with state of the art xylanases used to make prebiotics from AX clearly show the difference in hydrolysis products obtained (Figure 1 and Table 1). Production of AXOS is further optimized by choosing an A/X ratio in the interval of 0.2-0.7 preferably 0.28-0.65, preferably 0.35-0.5, preferably 0.38-0.45, preferably 0.4. The AXOS preparations are particularly useful as selective prebiotics for certain groups of intestinal bacteria belonging to the group of bifidobacteria adapted to ferment AXOS or the arabinose substituents attached to the AXOS molecule(s).

**Table 1. Peak retention times for Figure 1**

| | Retention time (min) | | |
|---|---|---|---|
| Peak number | *Rm*Xyn10A - - GH10 | Arabinoxylanase - GH5 | Pentopan - GH11 |
| 1 | 2.659 (Xylose) | 3.033 | 2.667 (Xylose) |
| 2 | 3.684 (Xylobiose) | 3.317 | 3.700 (Xylobiose) |
| 3 | 4.284 | 4.683 | 5.609 (Xylotriose) |
| 4 | 5.617 | 6.867 | 11.059 |
| 5 | 11.342 | 8.325 | 12.759 |
| 6 | 12.109 | 9.675 | 13.325 |
| 7 | 12.309 | 10.95 | 14.075 |
| 8 | 13.117 | 11.442 | 14.592 |
| 9 | 18.509 | 11.942 | 19.284 |
| 10 | 20.242 | 12.8 | 20.342 |
| 11 | 20.892 | 13.475 | 21.475 |
| 12 | 21.409 | 14.075 | 22.325 |
| 13 | 21.909 | 14.817 | 23.209 |

| | | | |
|---|---|---|---|
| Note: Xylo-oligosaccharide strandards retention time: Xylose: 2.667; Xylobiose: 3.700; Xylotriose: 5.600; Xylotetraose: 7.825; Xylopentaose: 9.359 and Xylohexaose: 10.384. | | | |

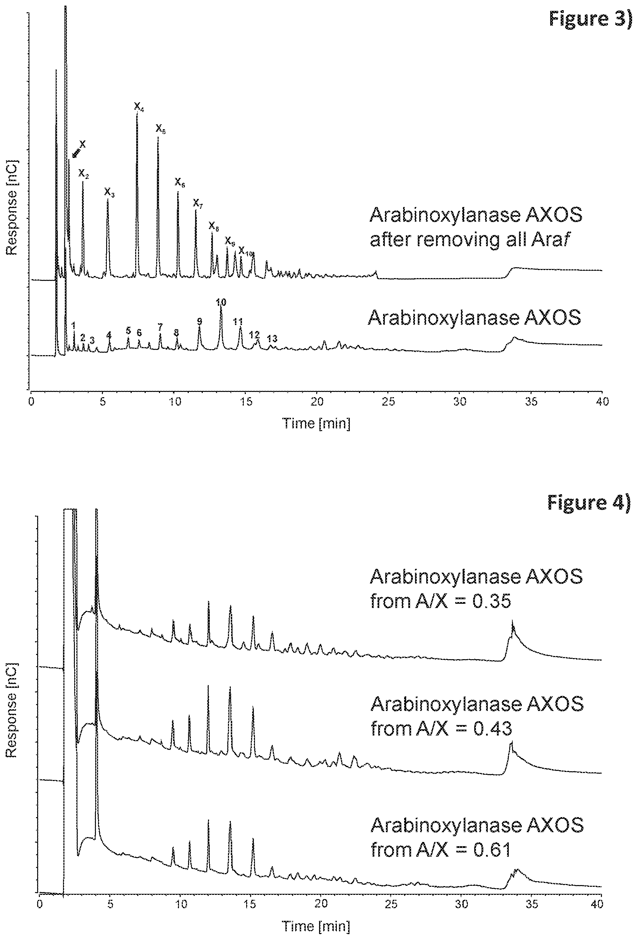

In the first example AXOS are generated from endosperm (flour) AX from but not limited to wheat and rye. The endosperm AX is optionally enzymatically treated with arabinofuranosidases to remove a fraction of the Araf groups in order to improve the yield of AXOS. Pure AXOS generated from endosperm AX is shown in Figure 2 for wheat and in Figure 3 for rye. This data shows that there are no xylose or XOS formed in the preparations (less than 0.1% on dry weight basis). Only after removing all Ara*f* groups attached to the obtained AXOS with an arabinofuranosidase or hydrochloric acid are the xylose and XOS backbones exposed. This confirms that all the obtained oligosaccharides are AXOS with no or very little formation of xylose and XOS. From the analysis of the XOS backbone after removing all Ara*f* groups it is determined that the backbone degree of polymerisation (DP) of the AXOS is 1-8 for wheat endosperm AX (Figure 2) and 1 - 10 for rye endosperm AX (Figure 3). The majority of AXOS from wheat and from rye endosperm AX had an average backbone of 3 (Table 2).

**Table 2. Xylose and XOS content in arabinofuranosidase or HCI treated AXOS samples from wheat and rye respectively presented as molar percentage**

| XOS backbone | Wheat endosperm AX (A/X 0.43) | Rye endosperm AX (A/X 0.64) |
|---|---|---|
| Xylose | 16% | 20% |
| Xylobiose | 10% | 15% |
| Xylotriose | 29% | 17% |
| Xylotetraose | 26% | 23% |
| Xylopentaose | 13% | 16% |
| Xylohexaose | 6% | 10% |

Further was the impact of the arabinose content in the AX substrate determined for the generation of AXOS by an arabinoxylanase. The highest yield of AXOS obtained from AX was achieved using an A/X of 0.43 (Figure 4) demonstrating that the optimal generation of AXOS is in the interval of A/X = 0.61-0.35 with 0.43 closest to the optimal. Significance of this is in the optimized production of AXOS by partially removing Ara*f* groups from the AX substrate prior or during the arabinoxylanase treatment.

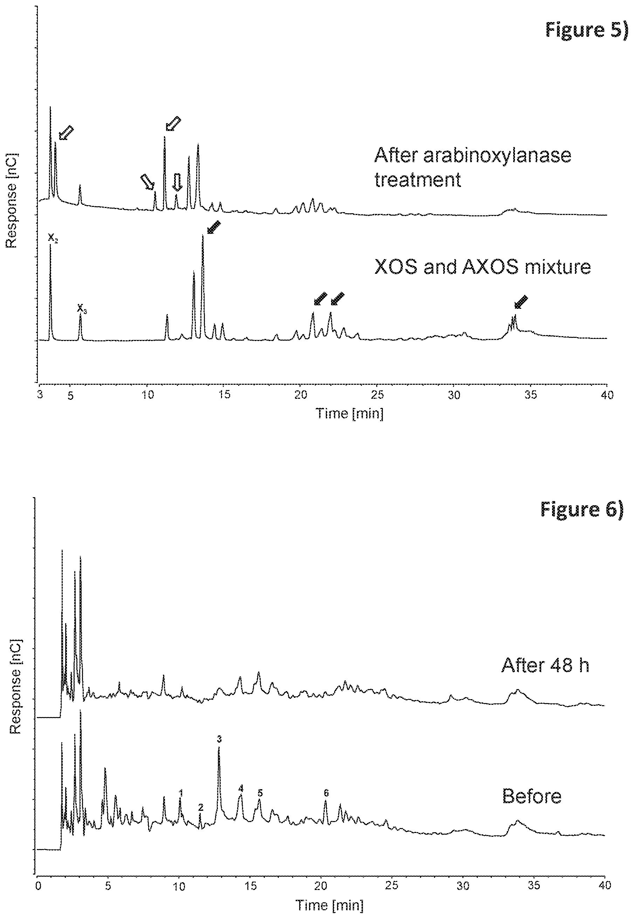

Another application of the technology is demonstrated in improved generation of AXOS in mixtures containing both XOS and AXOS. By adding an arabinoxylanse to an (A)XOS mixture from a family 11 xylanase new AXOS are formed by degrading poly- and oligosaccharides not hydrolysed by the family 11 xylanase (Figure 5). These XOS and AXOS mixtures can be obtained using state of the art xylanase treatments and the addition of the arabinoxylanase is a way to improve the generation AXOS in such preparations. Preparations similar to the ones mentioned in EP 2 265 127 B1 to improve the prebiotic properties of such products containing XOS and AXOS.

In the second example wheat bran is used as a substrate to make different fractions of AX suitable for making AXOS by an arabinoxylanase. The fractions are isolated from bran material by first removing starch and proteins followed by an extraction of the AX components from the bran material. The AX is then subsequently treated enzymatically with an arabinofuranosidase, or acid treated to obtain fractions with different A/X ratios (Table 3) that could be used to make different AXOS compositions using an arabinoxylanase.

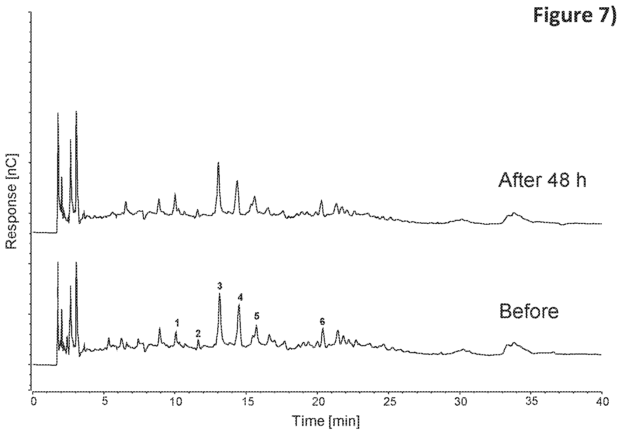

In the third example it is demonstrated that the obtained AXOS can be used to selectively stimulate the growth of bifidobacteria over other groups of intestinal bacteria that normally can use xylose or XOS (Figure 6 and 7). The reason is that the AXOS obtained do not contain neither xylose nor XOS that normally could be utilized by e.g. *Lactobacillus brevis.* There is also a difference in the carbohydrate preferences between different bifidobacteria strains which means that the AXOS can be used to stimulate certain strains of bifidobacteria. Strains from either cluster II, III, IV and V could be selectively stimulated with preparations containing only AXOS.

Representative strains from cluster II-V are but not limited to the following strains of bifidobacteria:
- *Bifidobacterium longum* subsp. *longum* DSMZ 20219 (Cluster 2)
- *Bifidobacterium adolescentis* DSMZ 20083 (Cluster 3)
- *Bifidobacterium longum* subsp. *longum* CCUG 15137 (Cluster 4)
- *Bifidobacterium catenulatum* DSMZ 16992 (Cluster 5)

Especially strains belonging to cluster 4 and 5, are able to efficiently utilize the entire AXOS and are of special interest to combine with the obtained AXOS. However, all bifidobacteria, able to cleave the arabinose substituents present on AXOS or utilize the entire AXOS, are possible to stimulate.

### Examples

### Example 1: Preparation of arabinoxylanase AXOS

### Materials and methods

Arabinoxylanase from *Clostridium thermocellum* (CtXyl5A) was purchased from Nzytech (Lisboa, Portugal). A family 10 xylanase from *Rhodothermus marinus* (*Rm*Xyn10A) was prepared as described in Falck et al. (2013). Pentopan mono bg, a commercial family 11 xylanase was obtained from Novozymes (Bagsvaerd, Denmark). High purity recombinant α-L-arabinofuranosidase (E-ABFCJ) from *Cellvibrio japonicus* was purchased from Megazyme (Wicklow Ireland). Endosperm AX extracted by alkali from wheat (P-WAXYM, P-EDWAX30, P-ADWAX26, P-ADWAX22) and rye (P-RAXY) were purchase from Megazyme. AX substrates were dissolved 10 g/L according to manufactures instructions in 50 mL MQ water and the pH was adjusted to 7 with 8M HCl. Arabinoxylanase from family 5 and xylanases from family 10 and 11 were added at an enzyme to substrate ratio of 1:1000 on a mass basis. In the arabinoxylanase reactions 2 mM CaCl₂ was used to stabilize the enzyme. All reactions were performed at 50°C for 24h using either a thermoblock or water bath. Enzymes were inactivated by incubating the sample at 95°C for 30 minutes.

The comparison between the arabinoxylanase and family 10 and 11 xylanases (Figure 1) was performed using wheat endosperm AX (P-EDWAX30) with 30% arabinose content equal to an A/X of 0.43. The AX had been treated with an arabinofuranosidase from *Bacteroides ovatus* to remove all α-(1→3)-linked Ara*f* at double substituted Xyl*p* units (dXyl). Characterization of the AXOS backbones of xylose and XOS in the arabinoxylanase sample (Figure 2) was performed with 0.5 U/(mg AXOS) using an arabinofuranosidase (E-ABFCJ) removing Ara*f* from (1→2) or (1→3) single substituted Xyl*p* units, mXyl2 and mXyl3 respectively. The reaction was performed at pH 5.8 using a 20 mM sodium phosphate buffer at 50°C for 24h. The treatment resulted in a complete removal of Ara*f* from the AXOS (Figure 2). Rye endosperm AX was treated in the same way as wheat with the exception that the arabinoxylanase reaction was performed for 48 hours. To remove all single and double substituted Ara*f* groups from the obtained AXOS a weak acid treatment was used. The pH was set to 2.8 with a diluted HCI solution and the sample (5 mL) was incubated at 90°C for 24h resulting in an almost complete removal of all Ara*f* groups from the AXOS (Figure 3).

The relation between arabinose content and the yield of arabinoxylanase generated AXOS was determined using wheat endosperm with different arabinose content. P-WAXYM, P-EDWAX30, P- ADWAX26 and P-ADWAX22 with an arabinose content of 38 %, 30%, 26% and 22% percent respectively or based on A/X 0.61, 0.43, 0.35 and 0.28 respectively (Figure 4). The reactions were performed in glass vials using 2 mL reaction volumes and a substrate concentration of 0.2 g/L substrate concentration. Arabinoxylanase was used to treat pentopan generated (A)XOS to generate more and shorter AXOS in the XOS and AXOS mixtures (Figure 5) using the same reaction conditions as described previously for the xylanase reactions.

### Characterization of AXOS and XOS

Analysis of the obtained AXOS fractions and XOS backbones was done by High-Performance Anion-Exchange Chromatography Coupled with Pulsed Electrochemical Detection (HPAEC-PAD) using (ICS-5000) using a CarboPac PA200 column (250 mm x 3 mm, 5.5 µm) and a guard column (50 mm x 3 mm) of the same material and a mobile phase of 100 mM NaOH at 0.5 mL/min and a linear gradient (0-30 min) of 0-120 mM of sodium acetate (Sigma). Monosaccharide and xylooligosaccharide standards used were as follows: arabinose and xylose (Sigma), xylobiose, xylotriose, xylotetraose, xylopentaose and xylohexaose (Megazyme). All samples were filtered through a 0.22 µm filter and diluted to a final concentration of 0.2 g/L before analysis.

### Example 2: Preparation of AX substrates with different A/X ratios from bran Materials and methods

Commercial wheat bran (Lantmännen Mill Malmö, Sweden) was used as starting material for the preparation of AX with different arabinose content defined as A/X. A suspension (1:9 w/v) of 250 g wheat bran in 2.5 L DI water was adjusted to pH 6.0 with HCI 8 M and treated with a thermostable α-amylase 0.12 U/g (Thermamyl, SIGMA-ALDRICH) for 90 min at 90°C to hydrolyse the starch. The bran was then rinsed with hot tap water to remove solubles until a clear permeate was obtained. A new suspension in water (1:9 w/v) was prepared to remove proteins by incubating with a protease 0.035 U/g (Neutralse 0.8L, SIGMA-ALDRICH) for 4h at 50°C. Thereafter the bran was rinsed with hot tap water, then with DI water and then vacuum dried. Destarched and deproteinised wheat bran was extracted with a dilute alkaline solution (NaOH) of hydrogen peroxide containing 2% hydrogen peroxide at pH 11.5 for 4h at 60°C under 200rpm stirring to obtain soluble AX. Antifoam TRITON X-100 was added to reduce foaming. After the extraction solids were removed by filtration and the solution was centrifuged (SIGMA) 6000g for 20 min. The supernatant was neutralized with 8 M HCI and horseradish peroxidase was added to remove remaining hydrogen peroxide. The extract was centrifuged again at 6000g for 20 minutes. The supernatant was divided and 50 mL was adjusted to pH 6 with 8M HCI and treated with 5 U of an arabinofuranosidase from *Bifidobacterium adolescentis* (Megazyme, E-AFAM2) by incubating the sample at 37°C for 24h. Supernatant was also acid debranched by a weak HCI acid at pH 2.5 at 90°C on a magnetic plate stirrer at 200 rpm. Samples (50 mL) were removed and neutralized with 1M NaOH after 3.4, 5.1, 6.8, and 8.6h. All fractions were desalted by dialysis bags (SpectrumLab, USA) using a 3500 Da Mw cut off. Dialysis was performed in 5L DI water twice and then all samples were freeze dried.

### Characterization of the isolated preparations

The monosaccharide composition of the AX fractions were analysed by HPAEC-PAD after hydrolysing the samples with 2 M TFA for 60 min at 110°C. Total arabinoxylan content in the samples were calculated as 0.88 times (% arabinose + % xylose) after subtracting any free arabinose. Analysis of the obtained monosaccharides was done by HPAEC-PAD using a CarboPac PA20 column (250 mm x 3 mm, 5.5 µm) and a guard column (30 mm x 3 mm) of the same material and a mobile phase of 0.75 mM NaOH at 0.5 mL/min with a post column addition of base of 100 mM at 0.15 mL/min. Monosaccharide (SIGMA) were as follows: arabinose, galactose, glucose and xylose. The resulting A/X fractions obtained are listed in Table 3.

**Table 3. Carbohydrate composition (w/w of total carbohydrates) and A/X of AX fractions obtained from wheat bran**

| Fraction | Arabinose | Glucose | Xylose | A/X |
|---|---|---|---|---|
| Supernatant | 0.42 | 0.02 | 0.57 | 0.73 |
| Abf. | 0.38 | 0.02 | 0.60 | 0.63 |
| HCl 3.4 h | 0.37 | 0.02 | 0.61 | 0.60 |
| HCl 5.1 h | 0.33 | 0.02 | 0.64 | 0.52 |
| HCl 6.8 h | 0.31 | 0.02 | 0.67 | 0.47 |
| HCl 8.6 h | 0.29 | 0.02 | 0.69 | 0.41 |

| | | | | |
|---|---|---|---|---|
| Note: Abf - arabinofuranosidase treated sample | | | | |

### Example 3: Selective growth of intestinal bacteria on arabinoxylanase AXOS Materials and methods

The bacterial strains used to test the fermentability of the obtained AXOS from rye endosperm AX were *Bifidobacteria adolescentis (B. adolescentis)* ATCC 15703 and *Lactobacillus brevis* (*L. brevis*) DSMZ 1269. *B. adolescentis, L. brevis,* were all pre-cultivated twice using 5 g/L glucose as carbon source. *B. adolescentis* was inoculated in *Bifidobacterium* medium at 37 °C and pH 6.8. The medium contained 12.5 g of casein peptone, tryptic digest, 6.25 g of yeast extract, 6.25 g of meat extract, 6.25 g of bacto soytone, 2.5 g of K₂PO₄, 0.25 g of MgSO₄·7H₂O, 0.0625 g of MnSO₄·H₂O, 6.25 g of NaCl, and 1.25 mL of Tween 80 per litre, respectively. To this solution was added 5 mL of solution with resazurin (25 mg/100 mL) together with 50 mL of salt solution containing 0.25 g of CaCl₂·H₂O, 0.5 g of MgSO₄·7 H₂O, 1 g of K₂HPO₄, 1 g of KH₂PO₄, 10 g of NaHCO₃, and 2 g of NaCl per litre, respectively. The medium was subsequently boiled followed by cooling under N₂ gas. Cysteine was added to a concentration of 0.625 g/L and adjusted to pH 6.8 using NaOH. *L. brevis* was grown anaerobically in MRS broth at pH 6.5 under anaerobic condition at 37 °C. All media for the cultivation experiments, broth as well as agar, were autoclaved at 121 °C for 15 min. All cultivation media used for anaerobic growth were deaerated by replacing the oxygen in the anaerobic tubes with nitrogen gas. Then, all tubes were closed with metal caps and autoclaved at 121 °C for 15 min. The respective carbon sources glucose and AXOS were filter sterilized through a 0.45 µm filter and added to the media at a final concentration of 5 g/L and a total volume of 5 mL. The fermentation experiment started from the second pre-culture using 2% vol. /vol. inoculum and samples were withdrawn after 24 and 48h. Optical density and pH was measured after 0, 24 and 48h, while consumption of oligosaccharides was analysed after 48 hours using HPAEC-PAD with the same conditions as described for the oligosaccharide analysis. *B. adolescentis* could grow on the arabinoxylanase AXOS produced from rye endosperm AX while *L. brevis* could not due to the fact that the preparation does not contain any xylose or XOS molecules (Figure 6 and 7 respectively).

**Table 4. Net change in optical density and pH after 48 h**

| *B. adolescentis* | *L. brevis* |
|---|---|
| OD: 0.2 | OD: 0.0 |
| pH: 0.56 | pH: 0.0 |

### References

FALCK, P., PRECHA-ATSAWANAN, S., GREY, C., IMMERZEEL, P., STÅLBRAND, H., ADLERCREUTZ, P., NORDBERG KARLSSON, E. 2013. Xylooligosaccharides from hardwood and cereal xylans produced by a thermostable xylanase as carbon sources for Lactobacillus brevis and Bifidobacterium adolescentis. Journal of Agricultural and Food Chemistry 61, 30, 7333-7340.
SWENNEN, K., COURTIN, C. M., LINDEMANS, G. C., & DELCOUR, J. A. 2006. Large scale production and characterisation of wheat bran arabinoxylooligosaccharides. Journal of the Science of Food and Agriculture, 86, 1722-1731.

## Claims

1. Process for producing an arabinoxylo-oligosaccharide composition from flour comprising the steps of:
A. extracting and isolating an endosperm arabinoxylan fraction from flour;
B. optionally removing starch and proteins from the obtained product of step A;
C. optionally treatment of the endosperm arabinoxylan of step A or product of step B with arabinofuranosidases, preferably one able to remove α-(1→3)-linked L-arabinofuranosyl at double substituted β-(1→4)-linked D-xylopyranosyl units (dXyl);
D. adding an arabinoxylanase to the obtained product of step A, step B or step C; and
E. drying the obtained material of step D.

2. Process for producing an arabinoxylo-oligosaccharide composition from bran comprising the steps of:
A'. removal of starch and proteins from the bran;
B'. recovery of a solid phase from A';
C'. treating the solid phase with alkaline solution, alkaline and peroxide solution or treating the solid phase with heat to provide a soluble phase;
D'. neutralizing the soluble phase comprising arabinoxylan of C' and recovery of said soluble phase comprising arabinoxylan;
E'. removing arabinose from the arabinoxylan containing soluble phase in step D' using arabinofuranosidases or a weak acid solution to obtain a molar ratio of arabinose to xylose of 0.2-0.7, preferably 0.35-0.5, preferably 0.38-0.45, preferably 0.4;
F'. separation to recover the arabinoxylan obtained from step E', preferably by precipitation or membrane separation;
G'. adding an arabinoxylanase to the arabinoxylan from step F'; and
H'. drying the obtained material of step G'.

3. Process for producing an arabinoxylo-oligosaccharide composition according to claim 1 or 2, wherein the arabinoxylo-oligosaccharide composition is produced using an arabinoxylan specific endoxylanase, preferably wherein the arabinoxylan specific endoxylanase is arabinoxylanase.

4. Process for producing an arabinoxylo-oligosaccharide composition according to claim 2 or 3, wherein the step C' includes an optional treatment with arabinofuranosidases to increase the yield of arabinoxylo-oligosaccharides.

5. Process for producing an arabinoxylo-oligosaccharide composition according to any one of claims 2-4, wherein the step A' includes removal of starch and proteins with amylases and proteases respectively.

6. Process for producing an arabinoxylo-oligosaccharide composition according to any one of claims 2-5, wherein the step C' includes extraction with alkali and peroxide, with optional steam treatment to increase the water soluble arabinoxylan content.

7. Process for producing an arabinoxylo-oligosaccharide composition according to any one of claims 2-6, wherein the step E' includes an optional treatment with arabinofuranosidases or a weak acid to increase the yield of arabinoxylo-oligosaccharides.

8. Use of an arabinoxylanase for the generation of arabinoxylo-oligosaccharides in xylo-oligosaccharides and arabinoxylo-oligosaccharides comprising preparations, preferably wherein the preparation of xylo-oligosaccharides and arabinoxylo-oligosaccharides is prepared using a family 11 xylanase, preferably wherein the arabinoxylanase is a xylanase belonging to glycoside hydrolase family 5.

9. Use of an arabinoxylanase according to claim 8, wherein the arabinoxylo-oligosaccharides are generated from a cereal fiber.

10. Use of an arabinoxylanase according to claim 8 or 9, comprising the steps of:
A'. removing starch and optionally proteins from a cereal fiber;
B'. recovering a solid phase from A';
C'. treating the solid phase from step B' with a xylanase able to hydrolyze water-insoluble arabinoxylan;
D'. adding an arabinoxylanase able to hydrolyze highly substituted arabinoxylan to step C' for the generation of arabinoxylo-oligosaccharides;
E'. recovering of said soluble phase from step D' comprising the oligosaccharides;
F'. optionally purifying said soluble phase from step E';
G'. concentrating or drying of soluble phase from step F';
preferably wherein the cereal fiber is derived from rye, maize, millets, rice, barley, oat or wheat.

## Patentansprüche

1. Verfahren zur Herstellung einer Arabinoxylooligosaccharidzusammensetzung aus Mehl, welches die folgenden Schritte umfasst:
A. Extrahieren und Isolieren einer Endospermarabinoxylanfraktion aus Mehl,
B. gegebenenfalls Entfernen von Stärke und Proteinen aus dem erhaltenen Produkt von Schritt A,
C. gegebenenfalls Behandlung des Endospermarabinoxylans von Schritt A oder des Produkts von Schritt B mit Arabinofuranosidasen, vorzugsweise einer, die dazu fähig ist, α=(1→3)-gebundenes L-Arabinofuranosyl an doppelt substituierten β-(1→4)-gebundenen D-Xylopyranosyleinheiten (dXyl) zu entfernen,
D. Zugabe einer Arabinoxylanase zu dem erhaltenen Produkt von Schritt A, Schritt B oder Schritt C und
E. Trocknen des erhaltenen Materials von Schritt D.

2. Verfahren zur Herstellung einer Arabinoxylooligosaccharidzusammensetzung aus Kleie, welches die folgenden Schritte umfasst:
A'. Entfernen von Stärke und Proteinen von der Kleie,
B'. Isolieren einer festen Phase aus A',
C'. Behandeln der festen Phase mit alkalischer Lösung, alkalischer und Peroxidlösung oder Behandeln der festen Phase mit Hitze unter Bereitstellung einer löslichen Phase,
D'. Neutralisieren der Arabinoxylan umfassenden löslichen Phase von C' und Isolieren der Arabinoxylan umfassenden löslichen Phase,
E'. Entfernen von Arabinose aus der arabinoxylanhaltigen löslichen Phase aus Schritt D' unter Verwendung von Arabinofuranosidasen oder einer schwach sauren Lösung unter Erhalt eines Molverhältnisses von Arabinose zu Xylose von 0,2-0,7, vorzugsweise 0,35-0,5, bevorzugt 0,38-0,45, vorzugsweise 0,4,
F'. Auftrennung zur Isolierung des aus Schritt E' erhaltenen Arabinoxylans, vorzugsweise durch Fällung oder Membrantrennung,
G'. Zugabe einer Arabinoxylanase zu dem Arabinoxylan aus Schritt F' und
H'. Trocknen des erhaltenen Materials von Schritt G'.

3. Verfahren zur Herstellung einer Arabinoxylooligosaccharidzusammensetzung nach Anspruch 1 oder 2, wobei die Arabinoxylooligosaccharidzusammensetzung unter Verwendung einer arabinoxylanspezifischen Endoxylanase hergestellt wird, wobei es sich bei der arabinoxylanspezifischen Endoxylanase vorzugsweise um Arabinoxylanase handelt.

4. Verfahren zur Herstellung einer Arabinoxylooligosaccharidzusammensetzung nach Anspruch 2 oder 3, wobei der Schritt C' eine fakultative Behandlung mit Arabinofuranosidasen zum Erhöhen der Ausbeute an Arabinoxylooligosacchariden umfasst.

5. Verfahren zur Herstellung einer Arabinoxylooligosaccharidzusammensetzung nach einem Ansprüche 2-4, wobei der Schritt A' das Entfernen von Stärke und Proteinen mit Amylasen bzw. Proteasen umfasst.

6. Verfahren zur Herstellung einer Arabinoxylooligosaccharidzusammensetzung nach einem der Ansprüche 2-5, wobei der Schritt C' die Extraktion mit Alkali und Peroxid mit fakultativer Dampfbehandlung zum Erhöhen des Gehalts an wasserlöslichem Arabinoxylan umfasst.

7. Verfahren zur Herstellung einer Arabinoxylooligosaccharidzusammensetzung nach einem der Ansprüche 2-6, wobei der Schritt E' eine fakultative Behandlung mit Arabinofuranosidasen oder einer schwachen Säure zum Erhöhen der Ausbeute an Arabinoxylooligosacchariden umfasst.

8. Verwendung einer Arabinoxylanase zur Bildung von Arabinoxylooligosacchariden in Xylooligosacchariden und Arabinoxylooligosacchariden umfassenden Zubereitungen, wobei die Zubereitung von Xylooligosacchariden und Arabinoxylooligosacchariden vorzugsweise unter Einsatz einer Familie-11-Xylanase erfolgt, wobei es sich bei der Arabinoxylanase vorzugsweise um eine zur Glykosidhydrolasefamilie 5 zählende Xylanase handelt.

9. Verwendung einer Arabinoxylanase nach einem der Anspruch 8, wobei die Arabinoxylooligosaccharide aus einer Getreidefaser erzeugt werden.

10. Verwendung einer Arabinoxylanase nach Anspruch 8 oder 9, welche die folgenden Schritte umfasst:
A'. Entfernen von Stärke und gegebenenfalls Proteinen von einer Getreidefaser,
B'. Isolieren einer festen Phase aus A',
C'. Behandeln der festen Phase aus Schritt B' mit einer zur Hydrolyse von wasserunlöslichem Arabinoxylan fähigen Xylanase,
D'. Zugabe einer zur Hydrolyse von hochsubstituiertem Arabinoxylan fähigen Arabinoxylanase zu Schritt C' zur Erzeugung von Arabinoxylooligosacchariden,
E'. Isolieren der die Oligosaccharide umfassenden löslichen Phase aus Schritt D',
F'. gegebenenfalls Aufreinigen der löslichen Phase aus Schritt E',
G'. Einengen oder Trocknen der löslichen Phase aus Schritt F',
wobei die Getreidefaser vorzugsweise aus Roggen, Mais, Hirse, Reis, Gerste, Hafer oder Weizen gewonnen wird.

## Revendications

1. Procédé pour la production d'une composition d'arabinoxylo-oligosaccharide à partir de farine comprenant les étapes de :
A. extraction et isolement d'une fraction d'arabinoxylane d'endosperme de la farine ;
B. éventuellement élimination d'amidon et de protéines du produit obtenu de l'étape A ;
C. éventuellement traitement de l'arabinoxylane d'endosperme de l'étape A ou du produit de l'étape B avec des arabinofuranosidases, préférablement une capable d'éliminer le L-arabinofuranosyle lié en α-(1→3) au niveau de motifs D-xylopyranosyle (dXyl) liés en β-(1→4) doublement substitués ;
D. ajout d'une arabinoxylanase au produit obtenu de l'étape A, de l'étape B ou de l'étape C ; et
E. séchage du matériau obtenu de l'étape D.

2. Procédé pour la production d'une composition d'arabinoxylo-oligosaccharide à partir de son comprenant les étapes de :
A'. élimination d'amidon et de protéines du son ;
B'. récupération d'une phase solide de A' ;
C'. traitement de la phase solide avec une solution alcaline, avec une solution alcaline et de peroxyde ou traitement de la phase solide avec de la chaleur pour donner une phase soluble ;
D'. neutralisation de la phase soluble comprenant l'arabinoxylane de C' et récupération de ladite phase soluble comprenant l'arabinoxylane ;
E'. élimination d'arabinose de la phase soluble contenant de l'arabinoxylane dans l'étape D' à l'aide d'arabinofuranosidases ou d'une solution d'acide faible pour obtenir un rapport molaire d'arabinose à xylose de 0,2 à 0,7, préférablement de 0,35 à 0,5, préférablement de 0,38 à 0,45, préférablement 0,4 ;
F'. séparation pour récupérer l'arabinoxylane obtenu de l'étape E', préférablement par précipitation ou séparation avec une membrane ;
G' . ajout d'une arabinoxylanase à l'arabinoxylane de l'étape F' ; et
H'. séchage du matériau obtenu de l'étape G'.

3. Procédé pour la production d'une composition d'arabinoxylo-oligosaccharide selon la revendication 1 ou 2, la composition d'arabinoxylo-oligosaccharide étant produite à l'aide d'une endoxylanase spécifique pour l'arabinoxylane, préférablement l'endoxylanase spécifique pour l'arabinoxylane étant l'arabinoxylanase.

4. Procédé pour la production d'une composition d'arabinoxylo-oligosaccharide selon la revendication 2 ou 3, l'étape C' comprenant un traitement éventuel avec des arabinofuranosidases pour augmenter le rendement d'arabinoxylo-oligosaccharides.

5. Procédé pour la production d'une composition d'arabinoxylo-oligosaccharide selon l'une quelconque des revendications 2 à 4, l'étape A' comprenant l'élimination d'amidon et de protéines avec respectivement des amylases et des protéases.

6. Procédé pour la production d'une composition d'arabinoxylo-oligosaccharide selon l'une quelconque des revendications 2 à 5, l'étape C' comprenant une extraction avec un alcali et un peroxyde, avec un traitement éventuel avec de la vapeur pour augmenter la teneur en arabinoxylane soluble dans l'eau.

7. Procédé pour la production d'une composition d'arabinoxylo-oligosaccharide selon l'une quelconque des revendications 2 à 6, l'étape E' comprenant un traitement éventuel avec des arabinofuranosidases ou un acide faible pour augmenter le rendement d'arabinoxylo-oligosaccharides.

8. Utilisation d'une arabinoxylanase pour la génération d'arabinoxylo-oligosaccharides dans des préparations comprenant des xylo-oligosaccharides et des arabinoxylo-oligosaccharides, préférablement la préparation des xylo-oligosaccharides et des arabinoxylo-oligosaccharides étant préparée à l'aide d'une xylanase de la famille 11, préférablement l'arabinoxylanase étant une xylanase appartenant à la famille 5 de glycoside hydrolase.

9. Utilisation d'une arabinoxylanase selon l'une quelconque parmi la revendication 8, les arabinoxylo-oligosaccharides étant générées à partir d'une fibre de céréale.

10. Utilisation d'une arabinoxylanase selon la revendication 8 ou 9, comprenant les étapes de :
A'. élimination d'amidon et éventuellement de protéines d'une fibre de céréale ;
B'. récupération d'une phase solide de A' ;
C'. traitement de la phase solide de l'étape B' avec une xylanase capable d'hydrolyser un arabinoxylane insoluble dans l'eau ;
D'. ajout d'une arabinoxylanase capable d'hydrolyser un arabinoxylane hautement substitué à l'étape C' pour la génération d'arabinoxylo-oligosaccharides ;
E'. récupération de ladite phase soluble de l'étape D' comprenant les oligosaccharides ;
F'. éventuellement purification de ladite phase soluble de l'étape E' ;
G'. concentration ou séchage de la phase soluble de l'étape F' ;
préférablement, la fibre de céréale étant issue de seigle, de maïs, de millets, de riz, d'orge, d'avoine ou de blé.
